# EUROPEAN PATENT SPECIFICATION

(11) **EP 0 326 290 B1**
(45) Date of publication and mention of the grant of the patent: **27.12.1995**
(21) Application number: 89300511.6
(22) Date of filing: 19.01.1989
(51) Int. Cl.: A61N 1/38

(54) **Method and apparatus for applying asymmetric biphasic truncated exponential countershocks**
Verfahren und Apparat zur Anwendung von asymmetrischen, zweiphasigen, abgeschnittenen exponentiellen Gegenschocks
Procédé et appareil d'application de chocs opposés asymétriques biphasés tronqués exponentiels

(30) Priority: 19.01.1988 US 145515
(43) Date of publication of application: 02.08.1989
(73) Proprietor: TELECTRONICS N.V., Willemstad Curaçao (AN)
(72) Inventor: de Coriolis, Paul E., Miami Florida 33166 (US); Batty, John R., Miami Florida 33155 (US); Shook, Bruce J., North Andover Massachusetts 01845 (US)
(74) Representative: Sturt, Clifford Mark

(56) References cited:
- EP-A- 0 280 526
- EP-A- 0 281 219
- EP-A- 0 324 380
- US-A- 3 224 447
- US-A- 3 614 955
- US-A- 4 120 305
- US-A- 4 498 478
- US-A- 4 576 170
- US-A- 4 637 397
- CARDIOVASCULAR RESEARCH, Vol. 18, 1984 John C. SCHUDER et al. "Defibrillation of 100 kg calves with asymmetrical, bidirectional, rectangular pulses." pages 419-426
- JACC, Feb 1987, Vol. 9, No. 2, page 142A: WINKLE et al. "Improved Low Energy Defibrillation Efficacy in Man Using a Biphasic Truncated Exponential Waveform"

## Description

This invention relates to apparatus for applying countershocks to the heart. More particularly it relates to cardioverters and defibrillators which supply truncated exponential pulses, and to implantable devices of this type.

Ventricular arrhythmias are potentially lethal. In the instance of chaotic, non-coordinated muscle contraction, known as fibrillation, death can ensue within minutes after onset. To convert the fibrillation to an organized, slower cardiac rate, an electrical countershock is given. A high energy pulse of 400 joules or less is applied across the chest wall using an external defibrillator. However, such external defibrillators are located in hospitals and in rescue vehicles. Because death can ensue within ten minutes, medical assistance may arrive too late to resuscitate the patient.

For patients who have survived an episode of ventricular fibrillation, there is a high probability of reoccurrence. In addition, patients who have experienced sustained symptomatic ventricular tachycardia are at risk in that such arrhythmias may convert to fibrillation. It is these patients who benefit from an implantable cardioverter or defibrillator.

An implantable cardioverter or defibrillator must be capable of sensing ventricular cardiac electrical activity, of determining if the sensed electrical activity is ventricular tachycardia or fibrillation and of enabling a circuit which then delivers a high energy pulse to electrodes associated with the heart to perform cardioversion (a shock in synchronization with the cardiac cycle) or defibrillation.

In prior implantable defibrillators, a truncated exponential pulse (trapezoidal pulse) of 25 joules or more has been utilized. Such a trapezoidal pulse is produced by a few external defibrillators but, more commonly, a damped sine wave is used. External defibrillators required a higher energy source, of up to 400 joules, because of energy dissipation through the chest wall.

Schuder et al, in "Ultrahigh-Energy Hydrogen Thyratron/SCR Bidirectional Waveform Defibrillator," Med & Biol. Eng. & Comput. Vol. 20, pp. 419-424 (July, 1982), show a symmetrical bi-directional truncated exponential waveform having less than 10% droop, so as to approximate a rectangular waveform, and a schematic diagram of an apparatus for generating such a waveform. The bi-directional pulse as described by Schuder et al. is incorporated into a rather large external defibrillator. Such bi-directional pulses can defibrillate and do not appear to influence adversely the outcome of subsequent attempts to defibrillate. However, much of the energy stored in the capacitor is lost, because the charge on the capacitor at the end of each phase must be dumped when the voltage is still a large fraction of the initial charge voltage.

In an implantable defibrillator, it is necessary to conserve space so that the implantable unit is not large. Further, because battery energy is finite in an implantable unit, any increase in efficiency that can be attained by fuller utilization of the stored energy (as measured in joules) or reduction in defibrillation threshold is of critical importance. Such increase in efficiency extends the useful life of the implant, thus reducing the frequency of implant replacement and the cost associated with an inefficient device due to the cost of the device itself, and surgical and hospital expenses incurred as a result of replacement. While replacement is a rather simple procedure, the patient is nevertheless exposed to the risks inherent in any form of surgery. In addition, reduction in defibrillation threshold, while saving energy, also has the beneficial effect of reducing the discomfort experienced by the patient when the defibrillation shock is applied.

European Patent Specifications EP-A-O 324 380; EP-A-O 280 526; and EP-A-O 281 219 are each relevant to the present application under Article 54(3) EPC. Each one of these specifications relates to an apparatus for administering electrotherapy to a heart similar to the one defined in claim 1 of the present application.

JACC, Feb 1987, Vol. 9, No. 2, page 142A: WINKLE et al "Improved Low Energy Defibrillation Efficacy in Man Using a Biphasic Truncated Exponential Waveform" discloses an implantable defibrillator using a biphasic truncated exponential waveform.

The Paper entitled "Defibrillation of 100 kg calves with asymmetrical bidirectional, rectangular pulses" published in Cardiovascular Research, 1984, Vol. 18, pages 419-426 discloses an external defibrillator using an asymmetrical biphasic rectangular pulse.

Various aspects of the invention will now be described, without limiting the scope of the invention.

It is an object of the invention to provide a method and apparatus for applying electrotherapy to the heart which is energy efficient.

It is another object of the invention to provide a method an apparatus for defibrillating the heart at a relatively low defibrillation threshold.

It is an additional object of the invention to provide an apparatus for applying electrotherapy to the heart which may be implanted within the body.

It is a further object of the invention to provide a method and apparatus for applying biphasic countershocks to the heart by using a single capacitor or a single capacitor bank for energy storage.

It is still another object of the invention to provide a switching circuit which permits the use of a single capacitor bank in an apparatus and method for applying electrotherapy to the heart, which circuit minimizes voltage stresses on the components therein.

According to the present invention, there is provided an implantable apparatus for administering electrotherapy to a heart, comprising: a recognition means responsive to an electrical signal from the heart for determining when the heart is in need of electrotherapy; a capacitive energy storage means for storing electrical energy to be applied to the heart; an energy source means for providing said electrical energy to said energy storage means; conductor means for conducting said stored electrical energy from said energy storage means to the heart; and connection means for connecting said conductor means with a first polarity to administer a first shock, and with a second polarity opposite said first polarity to administer a second shock, alter administration of said first shock, said connection means including: a first switch means, including first and second switches in series, for connecting a first terminal of said energy storage means to a first lead to the heart, a unidirectional conducting means bridging the second series switch; a second switch means for connecting a second terminal of said energy storage means to said second lead to the heart; a third switch means for connecting said first terminal of said energy storage means to said second lead to the heart; a fourth switch means for connecting said second terminal of said energy storage means to a point between the first and second switches; and timing means for closing said first and second series switches and said second switch means at a first time, for opening said first and second series switches and said second switch means at a second time alter said first time, for closing said third switch means and said fourth switch means at a third time alter said second time, and for opening said third switch means while closing said first series switch at a fourth time alter said third time thereby terminating said second polarity second shock, said first series switch and said fourth switch means being adapted to discharge said capacitive energy storage means at said fourth time.

According to the present invention, there is also provided a method for operating an implantable apparatus to supply electrical current to a heart by way of first and second conductive leads electrically connected to electrodes associated with the heart, the apparatus including a capacitor and a switch connection means including:
a first switch means, including first and second switches in series, for connecting a first terminal of the capacitor to the first conductive lead, a unidirectional conducting means bridging the second series switch;
a second switch means for connecting a second terminal of the capacitor to the second conductive lead;
a third switch means for connecting the first terminal of the capacitor to the second conductive lead;
a fourth switch means for connecting the second terminal of the capacitor to a point between the first and second switches;
wherein the method comprises the steps of:
applying a first truncated exponential waveform of a first polarity having a first start amplitude and a first end amplitude to the first and second conductive leads by charging the capacitor to a voltage corresponding to the first start amplitude, closing the first and second series switches and closing the second switch means at a first time to provide the first polarity signal to the heart and discharging the capacitor through the leads to a voltage corresponding to the first end amplitude;
applying to the leads a second truncated exponential waveform having a second start amplitude substantially equal to one half of the first start amplitude and a second end amplitude and having a second polarity opposite that of the first polarity, by reversing the polarity of connection of the capacitor to the leads by opening the first and second series switches and the second switch means at a second time alter the first time, closing the third and fourth switch means at a third time alter the second time to provide the second polarity signal to the heart thereby discharging the capacitor to a voltage corresponding to the second end amplitude, and shunting the charge of the capacitor at a fourth time after the third time by opening the third switch means while closing the first series switch.

According to the method embodying the invention, electrotherapy is applied to the heart by way of conductive leads electrically connected to electrodes associated with the heart. The leads and electrodes conduct pulses of electric current to the heart. These pulses include a first truncated exponential waveform of a first polarity having a first start amplitude and a first end amplitude; and a second truncated exponential waveform of a second polarity opposite that of said first polarity. The second truncated exponential waveform has a second start amplitude and a second end amplitude. The second start amplitude is lower than the first start amplitude. The second start amplitude may be substantially equal to the first end amplitude. Preferably the second start amplitude is equal to substantially one-half of the first start amplitude or is equal to between forty and sixty percent of the first start amplitude. The first truncated exponential waveform and the second truncated exponential waveform are applied by charging a capacitor to a voltage corresponding to the first start amplitude, discharging the capacitor through the leads to a voltage corresponding to the first end amplitude, reversing polarity of connection to the leads and discharging the capacitor to a voltage corresponding to the second end amplitude. The capacitor may be disconnected from the leads for a predetermined period of time before reversing the polarity of connection of the capacitor to the leads.

Further, the apparatus preferably includes a voltage reducing means for reducing the voltage across the fourth switch means at a fifth time, the fifth time being alter the second time and before the third time. The voltage reducing means shifts the voltage from across the fourth switch means so that it is across the first switch means. When the first switch means includes two switches connected in series, the apparatus may further comprise a first resistor connected across a first of the two switches, a fifth switch means having a first terminal connected to the second terminal of the energy storage means and a second resistor connecting a second terminal of the fifth switch means and a connection point between the first of the two switches and a second of the two switches. The apparatus further comprises a capacitor connected between the connection point and the second terminal of the energy storage means.

Further objects, features and advantages of our invention will become apparent upon consideration of the following detailed description in connection with the drawings, in which:
FIG. 1 is a simplified, schematic view along a section through a thorax showing an implantable defibrillator in accordance with the invention and its connection to leads associated with the heart;
FIG. 2 is a conceptual block diagram of the implantable defibrillator of FIG. 1;
Fig 3. is a conceptual schematic diagram of a circuit producing an asymmetric biphasic truncated exponential waveform with the polarity reversed;
FIG. 4 illustrates the waveform of the defibrillatory pulse of an implantable defibrillator in accordance with the invention;
FIG. 5 is a detailed schematic diagram of a circuit for use in the preferred embodiment of the apparatus according to the invention; and
FIG. 6A to FIG. 6I represent waveforms associated with the circuit of FIG. 5.

The invention is described herein with respect to an implantable defibrillator which is believed to be its primary, most important and most urgent area of application. However, it will be recognized in the art that the invention may be applied to external defibrillators and to cardioverters as well.

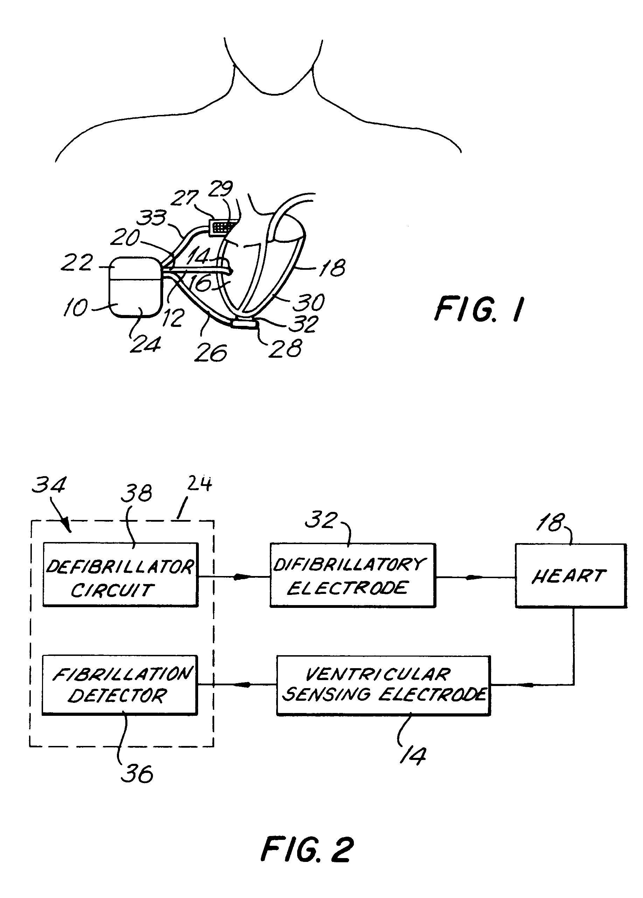

Referring to FIG. 1, an implantable defibrillator 10 is implanted subcutaneously in a patient, generally in an abdominal muscle. An epicardial sensing lead 12 including an insulated conductor wire is in electrical continuity with a ventricular sensing electrode 14 used for sensing ventricular electrical activity. Alternatively a pervenous sensing lead may be threaded through an appropriate vein and positioned in the right ventricle 16 of the heart 18. A terminal assembly (not shown) at the proximal end 20 of sensing lead 12 is inserted into a first receptacle (not shown) within the neck 22 of implantable defibrillator 10. The terminal assembly of sensing lead 12, when inserted in its respective receptacle, is electrically connected to the insulated conductor and to the circuitry of the defibrillator 10. This circuitry is encapsulated in a titanium case 24. Also encapsulated in case 24 is a low impedance battery, using lithium vanadium pentoxide or lithium silver vanadium pentoxide chemistry.

A terminal assembly (not shown) of a second lead 26 is connected to implantable defibrillator 10 via a second receptacle in neck 22 of defibrillator 10. The insulated conductor wire of lead 26 is tunneled subcutaneously. An electrode pad 28 at the distal end of lead 26 is sutured onto the epicardial surface of the left ventricle 30 of the heart 18 during a thoracotomy or median sternotomy. A subxephoid approach may also be used. A defibrillatory electrode 32, which acts as a cathode for the delivery of the first phase of biphasic defibrillation shocks, protrudes slightly from the cardiac surface of electrode pad 28 of lead 26.

A base patch 27 having an electrode 29 which is preferably epicardial, but as noted below may be subcutaneous, located at the distal end of a third lead 33, has a proximal terminal received in a third receptacle (not shown) in neck 22 of defibrillator 10. Electrode 29 acts as an anode for the delivery of the first phase of biphasic defibrillation shocks.

While lead 26 with its electrode 32 and lead 33 with its electrode 29 are epicardial,either lead may be epicardial or pervenous. Of importance, it is also contemplated that for defibrillation one lead and electrode may be pervenous, while the other may be a subcutaneous patch. The increased efficiency of defibrillation of an asymmetric biphasic truncated exponential pulse in accordance with the invention would thus permit the installation of an implantable defibrillator without necessitating opening the chest cavity. This greatly increases the desirability of the apparatus according to the invention by reducing the risk of patient morbidity or mortality as a result of risks associated with a more extensive surgical procedure.

Referring to FIG. 2, the circuitry 34 within the titanium case 24 is comprised of a fibrillation detector 36 (or a tachycardia detector for purposes of cardioversion) which may include any of several well-known arrhythmia detectors and a countershock or defibrillation circuit 38. Electrical activity generated by the ventricles 16,30 of the heart 18 is transmitted by the ventricular sensing electrode 14 to the fibrillation detector 36. If the detected ventricular activity is classified as fibrillation, as indicated by a chaotic, non-rhythmic electrical activity, defibrillator circuit 38 is enabled.

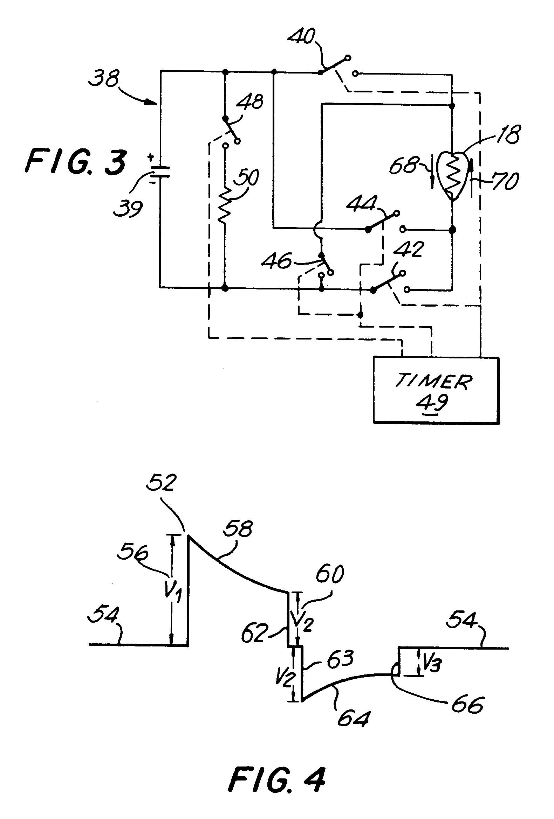

Referring to FIG. 3, the pulse forming or defibrillator circuit 38 is, in simplified or conceptual form, comprised of a capacitor 39, a series of switches 40, 42, 44, and 46, which appropriately open and close in response to signals from a timer circuit 49 (as described below, also with reference to FIG. 4), to deliver defibrillation shocks to the heart 18, and optionally, a switch 48 and a resistive load 50.

Referring to FIG. 4, an asymmetric biphasic exponential waveform 52, as recorded between defibrillatory electrodes 29 and 32, is measured from a baseline 54. Waveform 52 has a leading edge 56 of amplitude V1. The leading edge is followed by an exponential decay 58 of 2 to 8 milliseconds (for a time which may preferably be equal to approximately 0.7 times the time constant of capacitor 39 and the defibrillation resistance of the heart) to a second voltage level V2, at trailing edge 60, which waveform 52 returns to baseline 54. A zero voltage period 62 of 0.1 to 5.0 milliseconds is followed by a polarity reversal. The voltage V2 is then applied to the heart at leading edge 63 and a second exponential decay 64 occurs, which is preferably of the same duration, as the first decay 58. The voltage falls to a third voltage level V3 at trailing edge 66, from which it returns to baseline 54.

Asymmetric biphasic exponential waveform 52 is generated by utilizing circuit 38 in the following manner: capacitor 39 is charged by an energy source (not shown in FIG. 3) to a voltage V1 of the polarity shown. Switches 40 and 42 are closed by timer circuit 49 placing capacitor 39 in series with the heart 18. Current flows through the heart 18 in the direction of the arrow 68 discharging the capacitor to voltage V2. Switches 44, 46 and 48 are open during this time. Then, capacitor 39 is disconnected from the heart 18 and the voltage level of capacitor 39 is held at V2 when timer circuit 49 causes switches 40 and 42 to open. After a short no current interval, current is then caused to flow through the heart 18 in the direction as shown by the arrow 70 when timer circuit 49 causes switch 44 and switch 46 to close, thereby discharging capacitor 39 to voltage V3. The second phase is truncated when timer circuit 49 causes switch 44 and switch 46 to open (all others having remained open). Optional switch 48 may then be closed causing capacitor 39 to discharge into resistive load 50.

Thus, the defibrillatory pulse is actually made up of two pulses of current which travel through the heart first in one direction and then in the opposite direction increasing the probability of depolarizing and then rendering refractory a larger volume of the cardiac muscle fiber. The refractoriness makes it unlikely for fibrillation to continue. However, if fibrillation does continue, the capacitor is recharged and an additional pulse or pulses having the waveform illustrated in FIG. 4 may be administered.

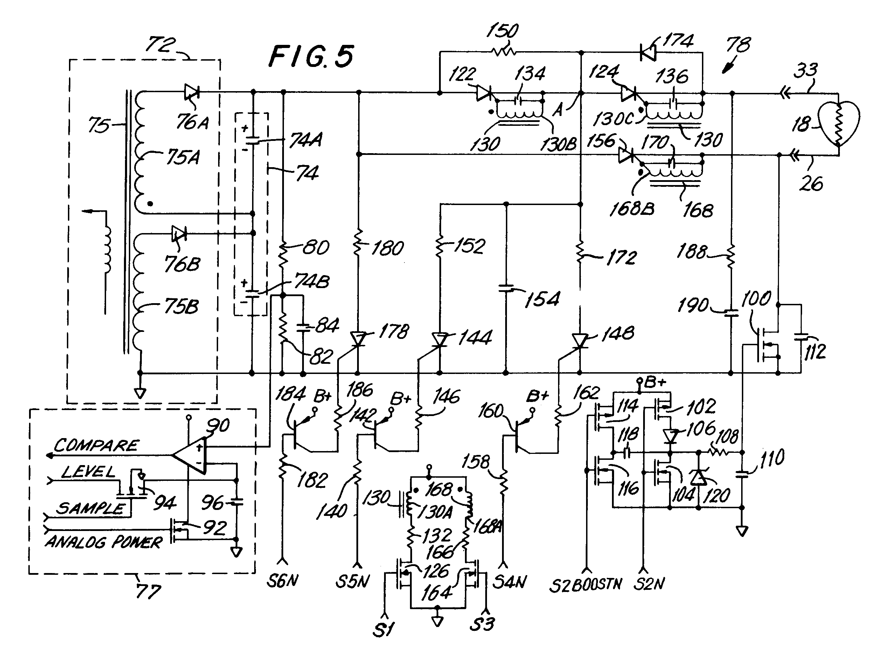

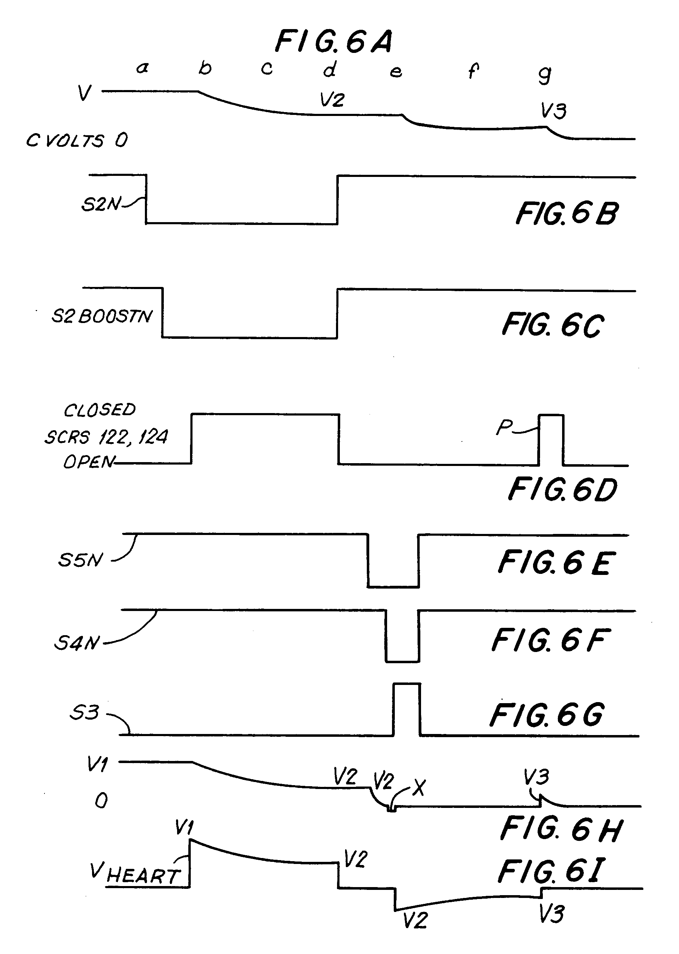

Referring to FIG. 5, the conceptual circuit of FIG. 3 is implemented. FIG. 5 includes a charging circuit 72 of conventional type for charging a single capacitor bank 74, a comparator circuit 77 for providing electrical signals indicative of the voltage to which capacitor bank 74 has been charged and a switching circuit shown generally at 78, which is a practical implementation of the concept of FIG. 3. While timer circuit 49 is not described, the necessary waveforms and their relationships in time are described below with respect to FIG. 6B to FIG. 6G.

When fibrillation is detected by fibrillation detector 36 (FIG. 2) capacitor bank 74 is charged by capacitor charging circuit 72. Charging circuit 72 may be any of several well-known circuits which have provisions for a "soft-start" so that the large current demands placed on the battery at the beginning of the charge cycle are somewhat reduced in magnitude and provisions for low battery voltage detection means for temporarily halting the charge cycle to permit battery recovery should the battery voltage drop to a predetermined low level.

While capacitor bank 74 would preferably include only a single capacitor, the limitations in capacitor technology, and in particular the limitations of aluminum electrolytic capacitors are such that the maximum working voltage is not sufficient to withstand the maximum voltage to which the capacitor must be charged. Thus, capacitor bank 74 may be made up of two capacitors 74A and 74B which are connected in series and charged by charging circuit 72. Specifically, capacitor 74A is charged by a secondary winding 75A of a step up transformer 75 through a diode 76A. In a similar manner, capacitor 74B is charged by current from secondary winding 75B of transformer 75 through diode 76B.

A voltage divider including resistors 80 and 82 and a smoothing capacitor 84 provide an output voltage to comparator circuit 77 which is proportional to, but a small fraction of, the voltage across capacitor bank 74.

Comparator circuit 77 includes an integrated circuit comparator 90. The positive supply voltage from the battery, appropriately increased in level by, for example, voltage doubling, is always supplied to circuit 90. However, the negative supply voltage to comparator 90 is connected through a MOSFET 92 which becomes conductive only upon application of an analog power enable signal to the gate thereof. Comparator 90 is thus turned off most of the time for purposes for saving power and turned on only during time periods when a voltage "measurement" must be made.

A sample-and-hold circuit including a MOSFET 94 and a capacitor 96 provides a reference voltage to one input of comparator 90. The other input of comparator 90 is connected to the junction between resistors 80 and 82. When the charging cycle is initiated, power is applied to comparator 90 through MOSFET 92 and a voltage level applied to the drain of MOSFET 94 is stored on capacitor 96 by supplying a sample pulse to the gate of MOSFET 94. It is desirable to use a sample-and-hold circuit, with the storage capacitor thereof located in close physical proximity to the comparator chip, and when in the hold mode, electrically isolated from the remainder of the circuitry of defibrillator 10, so that the large currents in the circuitry do not induce spurious voltages on the capacitor and detract from the accuracy of comparator circuit 77.

When capacitor bank 74 has charged to the appropriate voltage level, and the output of comparator 90 switches states, charging is complete. Charging circuit 72 is then turned off. A voltage representative of V2 is then loaded on to capacitor 96 of comparator circuit 77. A shock is initiated by operation of timer circuit 49 immediately for defibrillation or in synchronism with detected heart activity if cardioversion is being performed. However, it is desirable to reconfirm the presence of a tachyarrhythmia before timer circuit 49 operates to supply a shock.

During sensing and pacing, defibrillation lead 26 acts as a return for electrical conduction to the heart. The pacing and/or sensing signals from this lead are conducted through MOSFET 100 for this purpose. Thus, MOSFET 100 must be at least partially switched on at all times except during the charging of capacitor bank 74 at which time MOSFET 100 is switched off to provide protection against accidental discharge. MOSFET 100 is turned on by a switching circuit including MOSFETS 102 and 104. When the signal S2N (FIG. 6B) is at ground potential MOSFET 104 is off and MOSFET 102 is turned on. Diode 106 becomes conductive thus raising the potential of the gate of MOSFET 100 by conducting current through resistor 108. Capacitor 110 smoothes the voltage at the gate of MOSFET 100 and prevents transient impulses from turning on MOSFET 100. Capacitor 112 protects MOSFET 100 against currents due to electrosurgery by providing a bypass between the source and drain thereof.

While MOSFET 100 is held on so that its internal resistance is in the order of 20 to 30 ohms for sensing and pacing (when S2N is at logic zero), in order to conduct a defibrillation pulse MOSFET 100 must be put into a very low resistance condition or "turned on hard" so that its internal impedance is of a mere fraction of an ohm. This is accomplished through the action of MOSFETS 114 and 116 as well as capacitor 118. When S2BOOSTN from timer circuit 49 is at a logic high voltage level, MOSFET 114 is turned off and MOSFET 116 is turned on. Capacitor 118 is charged through diode 106 so that the side facing diode 106 is at a voltage close to that of the positive supply voltage (the doubled battery voltage). As shown in FIG. 6C, for the application of a defibrillation pulse, S2BOOSTN is dropped to ground potential by timer circuit 49. This turns off MOSFET 116 and turns on MOSFET 114. the result is to place capacitor 118 is series with the positive supply voltage so that aproximately twice that voltage is applied across the source and drain of MOSFET 104 (off at that time). This results in MOSFET 100 being turned on hard. Zener diode 120 serves to limit the voltage applied between the source and gate of MOSFET 100 so that it is not damaged. FIG. 6A illustrates that after capacitor bank 74 has been charged to voltage V1, S2N goes low and then S2BOOSTN goes low shortly thereafter (FIG. 6B and FIG. 6C). MOSFETS 102, 104, 114 and 118 may all be on a single type CD4007 chip.

In order to provide a defibrillation shock to the heart, it also necessary to turn on SCR 122 and SCR 124. This is accomplished when timer circuit 49 provides a pulse S1 (not shown in FIG. 6A to FIG. 6H) to the gate of MOSFET 126 completing a circuit through the primary winding 130A of transformer 130, current limiting resistor 132 and MOSFET 126. The surge of current through primary winding 130A induces a voltage in a first secondary winding 130B and a second secondary winding 130C of transformer 130. If the polarities of connection of the windings of transformer 130 are as indicated in FIG. 5, SCR 122 and SCR 124 are triggered. False triggering is prevented by capacitor 134 across secondary 130B and capacitor 136 across secondary 130C.

When MOSFET 100 and SCRs 122 and 124 have been turned on (the state of the latter two devices being represented by FIG. 6D) current is conducted through SCR 122, SCR 124, to a lead terminal in neck 22 of defibrillator 10, along defibrillation lead 33, through the heart 18, along defibrillation lead 26, to another lead terminal in neck 22 of defibrillator 10, and finally through MOSFET 100, all connected in series across capacitor bank 74.

After the output of comparator 90 has changed states due to the voltage across capacitor bank 74 being reduced to voltage V2, which may be some fixed fraction of the voltage V1, timer circuit 49 causes S2N and S2BOOSTN to return to a logic high state thus turning off MOSFET 100, commutating SCR 122 and SCR 124 (FIG. 6D) and terminating the initial phase of discharge of capacitor bank 74. At this point capacitor bank 74 is electrically disconnected from the heart.

For a period of time following the first phase, timer circuit 49 does not interact with the circuit of FIG. 5. In other words, there is a delay of 0.1 millisecond to 5 milliseconds during which no current is delivered to the heart 18. After this delay, capacitor bank 74 is reconnected to the heart with its polarity reversed. First, timer circuit 49 causes the voltage S5N (FIG. 6E) applied to resistor 140 to go from a positive logic level to zero volts (ground). Since the emitter of transistor 142 is connected to the positive supply voltage, this causes transistor 142 to turn on which in turn causes triggering of SCR 144 by way of current conducted through resistor 146. Before this occurs, the full voltage of capacitor bank 74 appears across SCR148. However, when SCR 144 is turned on much of this voltage is slowly shifted to appear across SCR 122 due to the voltage division action of resistor 150 and resistor 152. Typically, resistor 150 will have a resistance value at least 10 times as great as that of resistor 152. The voltage is gradually shifted due to the discharge time of a capacitor 154 through the series combination of resistor 152 and SCR 144. A practical time constant may be on the order of 10 to 50 microseconds. This shift in voltage is represented by the change in the voltage at point A (the connection point of SCR 122 and SCR 124) as shown in FIG. 6H. The slight jog in voltage at point X of FIG. 6H after the shift, occurs when SCR 148 is turned on as described below.

Timer circuit 49 causes the heart 18 to be connected to capacitor bank 74 by turning on SCR 148 and SCR 156. SCR 148 is triggered by changing the voltage S4N (FIG. 6F) applied to resistor 158 from a logic high state to a logic zero state (ground), thus turning on transistor 160 (the emitter of which is connected to the positive supply voltage) and triggering SCR 148 due to current conducted through resistor 162.

To turn on SCR 156 the voltage S3 at the gate at MOSFET 164 is changed from ground to a positive logic voltage (FIG. 6G). Current begins to flow through the series circuit including the primary winding 168A of a transformer 168, current limiting resistor 166 and MOSFET 164. The changing current in primary winding 168A induces a voltage in secondary winding 168B of transformer 168 thus triggering SCR 156. The capacitor 170 prevents false triggering of SCR 156 in a manner similar to that of capacitors 134 and 136.

The path of current from capacitor bank 74, at this time includes SCR 156, a lead connection terminal within neck 22 of defibrillator 10, defibrillation lead 26, the heart 18, defibrillation lead 33, a second lead terminal within neck 22 of defibrillator 10, a diode 174, a resistor 172 (which has a valve of no greater than several ohms) and finally SCR 148. Initially, voltage V2 (FIG. 4 and FIG. 6A) is applied to the heart and decays to voltage V3 due to discharge of capacitor bank 74. This second phase of the shock applied to the heart is truncated at a time so that its duration is preferably equal to the duration of the first phase (of opposite polarity) applied to the heart. Alternatively a third voltage representative of a desired value of V3 may be loaded on to capacitor 96 of comparator circuit 77, and a change in output of comparator 90 used to initiate truncation. To accomplish truncation, timer circuit 49 again supplies a short pulse to the gate of MOSFET 126, again triggering SCR 122 (as represented by pulse P, FIG. 6D). Capacitor bank 74 is thus discharged through SCR 122, resistor 172 and SCR 148.

It will be recognized that the start voltage of the second phase of the defibrillation shock will be slightly lower than that at the end of the first phase due to a small voltage drop across resistor 172. It is believed that voltages for V2 from sixty percent to forty percent of V1 produce the lowest defibrillation threshold. This voltage drop across resistor 172 is therefore inconsequential. It will be recognized that some resistance, rather than a simple short, is required to reduce current so as to protect SCR 122 and SCR 148 when capacitor bank 74 is being discharged.

An additional SCR 178, and a resistor 180 having a resistance of, for example, 50 ohms (which is comparable to the defibrillation path resistance of the heart) form a test load for the battery, capacitor charging circuit 72 and capacitor bank 74 and provide a means for dissipating the energy of capacitor bank 74 if charging is performed but no shock is applied to the heart. At appropriate times, as is well known in the art, a voltage S6N applied to a resistor 182 is changed from a positive logic voltage to ground thus turning on a transistor 184 having an emitter connected to the positive supply voltage and triggering SCR 178 when current is conducted through a resistor 186.

In summary, with reference to sequential points and intervals in time in FIG. 6A to FIG. 6H, as shown thereon:

Charging of capacitor bank 74 to voltage V1 occurs until point a.

Capacitor bank 74 is placed in series with the heart by turning on MOSFET 100 and then closing SCR 122 and SCR 124 simultaneously at point b.

Capacitor bank 74 is allowed to discharge to voltage V2 during interval c.

Capacitor bank 74 is disconnected from the heart by opening MOSFET 100, SCR 122 and SCR 124 at point d.

After a short delay, capacitor bank 74 is reconnected to the heart with inverted polarity by first closing SCR 144 to slowly shift voltage from SCR 148 to SCR 122 and then by closing SCR 148 and SCR 156 (the latter at point e).

Voltage V2 is applied to the heart and capacitor bank 74 is discharged to voltage V3 during interval f.

The second phase is truncated at V3 by closing SCR 122 which shunts charge in capacitor bank 74 away from the heart through the path defined by SCR 122, resistor 172 and SCR 148 during time interval g.

A major advantage of the circuit of FIG. 5 is that no SCR's are exposed to rapid positive voltage changes at any time. Resistor 150, resistor 152, capacitor 154 and SCR 144 preload SCR 122 into its blocking mode prior to application of voltage V2 to the heart. The elimination of rapid positive voltage changes makes possible the use of commonly available rugged and inexpensive switching components. For example, the SCRs which conduct defibrillation current may all be of type 2N6509, while MOSFET 102 may be a type IRF450. Building a more dependable and less complex energy delivery system by using such proven components is of major importance because in an implantable defibrillator any departure from high reliability would be anathema.

There are additional advantages to the circuit of FIG. 5. The circuit can be used to provide single monophasic pulses or sequential monophasic pulses by modifying the timing of appropriate control signals from timer circuit 49.

For additional protection against currents induced by electrosurgery a bypass circuit including resistor 188 and capacitor 190 may be provided.

Although the invention has been described with reference to a particular embodiment, it is to be understood that this embodiment is merely illustrative of the application of the principles of the invention. Numerous modifications may be made therein and other arrangements may be devised without departing from the scope of the invention.

## Claims

1. An implantable apparatus for administering electrotherapy to a heart, comprising:
a recognition means (36) responsive to an electrical signal from the heart for determining when the heart is in need of electrotherapy;
a capacitive energy storage means (74) for storing electrical energy to be applied to the heart;
an energy source means (72) for providing said electrical energy to said energy storage means;
conductor means (26, 33) for conducting said stored electrical energy from said energy storage means (74) to the heart; and
connection means (100, 122, 124, 148, 156) for connecting said conductor means (26, 33) with a first polarity to administer a first shock, and with a second polarity opposite said first polarity to administer a second shock, alter administration of said first shock, said connection means including:
a first switch means (122, 124), including first and second switches (122, 124) in series, for connecting a first terminal (+) of said energy storage means (74) to a first lead (33) to the heart, a unidirectional conducting means (174) bridging the second series switch;
a second switch means (100) for connecting a second terminal (-) of said energy storage means (74) to said second lead (26) to the heart;
a third switch means (156) for connecting said first terminal (+) of said energy storage means (74) to said second lead (26) to the heart;
a fourth switch means (148) for connecting said second terminal (-) of said energy storage means (74) to a point (A) between the first and second switches; and
timing means (49) for closing said first and second series switches (122, 124) and said second switch means (100) at a first time, for opening said first and second series switches (122, 124) and said second switch means (100) at a second time alter said first time, for closing said third switch means (156) and said fourth switch means (148) at a third time after said second time, and for opening said third switch means (156) while closing said first series switch (122) at a fourth time after said third time thereby terminating said second polarity second shock, said first series switch (122) and said fourth switch means (148) being adapted to discharge said capacitive energy storage means (74) at said fourth time.

2. The apparatus of claim 1, wherein said capacitive energy storage means (74) is a single capacitor (39).

3. The apparatus of claim 1, wherein the capacitive energy storage means (74) includes two capacitors (74A, 74B) connected in series, each of said two capacitors being of equal capacitance.

4. The apparatus of claim 1, further comprising voltage reducing means (144) for reducing the voltage across said fourth switch means at a fifth time, said fifth time being after said second time and before said third time.

5. The apparatus of claim 4, wherein said voltage reducing means (144) shifts said voltage from across said fourth switch means (148) to across said first series switch (122).

6. The apparatus of claim 1, further comprising a first resistor (150) connected across said first series switch (122), a fifth switch means (144) having a first terminal connected to the second terminal (-) of said energy storage means (74), and a second resistor (152) connecting a second terminal of said fifth switch means and a connection point between said first series switch (122) and said second series switch (124).

7. The apparatus of claim 6, further comprising a capacitor (154) connected between said connection point and said second terminal (-) for said energy storage means (74).

8. A method for operating an implantable apparatus to supply electrical current to a heart by way of first and second conductive leads (33, 26) electrically connected to electrodes associated with the heart, the apparatus including a capacitor (74) and a switch connection means including:
a first switch means, including first and second switches (122, 124) in series, for connecting a first terminal of the capacitor to the first conductive lead (33), a unidirectional conducting means (174) bridging the second series switch;
a second switch means (100) for connecting a second terminal of the capacitor (74) to the second conductive lead (26);
a third switch means (156) for connecting the first terminal of the capacitor to the second conductive lead (26);
a fourth switch means (148) for connecting the second terminal of the capacitor to a point (A) between the first and second switches;
wherein the method comprises the steps of:
applying a first truncated exponential waveform of a first polarity (58) having a first start amplitude (V1) and a first end amplitude (V2) to the first and second conductive leads by charging the capacitor to a voltage corresponding to the first start amplitude, closing the first and second series switches and closing the second switch means at a first time to provide the first polarity signal to the heart and discharging the capacitor through the leads to a voltage corresponding to the first end amplitude;
applying to the leads a second truncated exponential waveform having a second start amplitude (V2) substantially equal to one half of the first start amplitude (V1) and a second end amplitude (V3) and having a second polarity (64) opposite that of the first polarity, by reversing the polarity of connection of the capacitor to the leads by opening the first and second series switches (122, 124) and the second switch means (100) at a second time alter the first time, closing the third and fourth switch means at a third time alter the second time to provide the second polarity signal to the heart thereby discharging the capacitor to a voltage corresponding to the second end amplitude (V3), and shunting the charge of the capacitor at a fourth time after the third time by opening the third switch means while closing the first series switch (122).

9. A method according to claim 8, wherein said second start amplitude is equal to between forty and sixty percent of said first start amplitude.

## Patentansprüche

1. Implantierbare Vorrichtung zum Verabreichen von Elektrotherapie an ein Herz, mit:
einer Erkennungseinrichtung (36), die auf ein elektrisches Signal vom Herzen anspricht, zum Bestimmen, wann das Herz Elektrotherapie benötigt;
einer kapazitiven Energiespeichereinrichtung (74) zum Speichern von elektrischer Energie, die dem Herzen zuzuführen ist;
einer Energiequelleneinrichtung (72) zum Liefern der elektrischen Energie an die Energiespeichereinrichtung;
einer Leitereinrichtung (26, 33) zum Leiten der gespeicherten elektrischen Energie von der Energiespeichereinrichtung (74) an das Herz; und
einer Verbindungseinrichtung (100, 122, 124, 148, 156) zum Verbinden der Leitereinrichtung (26, 33) mit einer ersten Polarität, um einen ersten Schock zu verabreichen, und mit einer der ersten gegengesetzten zweiten Polarität, um nach Verabreichung des ersten Schocks einen zweiten Schock zu verabreichen, wobei die Verbindungseinrichtung aufweist:
eine erste Schaltereinrichtung (122, 124), mit einem ersten und einem zweiten Schalter (122, 124) in Reihe, zum Verbinden einer ersten Leitung (+) der Energiespeichereinrichtung (74) mit einer ersten Leitung (33) zum Herzen, wobei eine in einer Richtung leitende Einrichtung (174) den zweiten Reihenschalter überbrückt;
eine zweite Schaltereinrichtung (100) zum Verbinden des zweiten Anschlusses (-) der Energiespeichereinrichtung (74) mit der zweiten Leitung (26) zum Herzen:
eine dritte Schaltereinrichtung (156) zum Verbinden des ersten Anschlusses (+) der Energiespeichereinrichtung (74) mit der zweiten Leitung (26) zum Herzen;
eine vierte Schaltereinrichtung (148) zum Verbinden des zweiten Anschlusses (-) der Energiespeichereinrichtung (74) mit einem Punkt (A) zwischen dem ersten und dem zweiten Schalter; und
eine Zeitsteuereinrichtung (49) zum Schließen des ersten und des zweiten Reihenschalters (122, 124) und der zweiten Schaltereinrichtung (100) zu einer ersten Zeit, zum Öffnen des ersten und des zweiten Reihenschalters (122, 124) und der zweiten Schaltereinrichtung (100) zu einer zweiten Zeit nach der ersten Zeit, zum Schließen der dritten Schaltereinrichtung (156) und der vierten Schaltereinrichtung (148) zu einer dritten Zeit nach der zweiten Zeit und zum Öffnen der dritten Schaltereinrichtung (156) während des Schließens des ersten Reihenschalters (122) zu einer vierten Zeit nach der dritten Zeit, wodurch der zweite Schock mit der zweiten Polarität beendet wird, wobei der erste Reihenschalter (122) und die vierte Schaltereinrichtung (148) so eingerichtet sind, daß sie die kapazitive Energiespeichereinrichtung (74) zu der vierten Zeit entladen.

2. Vorrichtung nach Anspruch 1, wobei die kapazitive Energiespeichereinrichtung (74) ein einzelner Kondensator (39) ist.

3. Vorrichtung nach Anspruch 1, wobei die kapazitive Energiespeichereinrichtung (74) zwei Kondensatoren (74A, 74B) aufweist, die in Reihe geschaltet sind, wobei jeder der beiden Kondensatoren die gleiche Kapazität hat.

4. Vorrichtung nach Anspruch 1, ferner mit einer Spannungsverringerungseinrichtung (144) zum Verringern der Spannung über der vierten Schaltereinrichtung zu einer fünften Zeit, wobei die fünfte Zeit nach der zweiten Zeit und vor der dritten Zeit liegt.

5. Vorrichtung nach Anspruch 4, wobei die Spannungsverringerungseinrichtung (144) die Spannung von der vierten Schaltereinrichtung (148) zu dem ersten Reihenschalter (122) verschiebt.

6. Vorrichtung nach Anspruch 1, ferner mit einem ersten Widerstand (150), der zu dem ersten Reihenschalter (122) parallel geschaltet ist, einer fünften Schaltereinrichtung (144) mit einem ersten Anschluß, der mit dem zweiten Anschluß (-) der Energiespeichereinrichtung (74) verbunden ist, und einem zweiten Widerstand (152), der einen zweiten Anschluß der fünften Schaltereinrichtung und einen Verbindungspunkt zwischen dem ersten Reihenschalter (122) und dem zweiten Reihenschalter (124) verbindet.

7. Vorrichtung nach Anspruch 6, ferner mit einem Kondensator (154), der zwischen den Verbindungspunkt und den zweiten Anschluß (-) für die Energiespeichereinrichtung (74) geschaltet ist.

8. Verfahren zum Betreiben einer implantierbaren Vorrichtung, um an ein Herz mittels einer ersten und einer zweiten leitfähigen Leitung (33, 26), die mit Elektroden, die mit dem Herzen in Verbindung stehen, elektrisch verbunden sind, elektrischen Strom zu liefern, wobei die Vorrichtung einen Kondensator (74) und eine Schalterverbindungseinrichtung aufweist mit:
einer ersten Schaltereinrichtung, mit einem ersten und einem zweiten Schalter (122, 124) in Reihe, zum Verbinden eines ersten Anschlusses des ersten Kondensators mit der ersten leitfähigen Leitung (33), wobei eine in einer Richtung leitende Einrichtung (174) den zweiten Reihenschalter überbrückt;
einer zweiten Schaltereinrichtung (100) zum Verbinden eines zweiten Anschlusses des Kondensators (74) mit der zweiten leitfähigen Leitung (26);
einer dritten Schaltereinrichtung (156) zum Verbinden des ersten Anschlusses des Kondensators mit der zweiten leitfähigen Leitung (26);
einer vierten Schaltereinrichtung (148) zum Verbinden des zweiten Anschlusses des Kondensators mit einem Punkt (A) zwischen dem ersten und dem zweiten Schalter;
wobei das Verfahren die folgenden Schritte aufweist:
Anlegen einer ersten verkürzten exponentiellen Wellenform mit einer ersten Polarität (58) mit einer ersten Anfangsamplitude (V1) und einer ersten Endamplitude (V2) an die erste und die zweite leitfähige Leitung durch Laden des Kondensators auf eine Spannung, die der ersten Anfangsamplitude entspricht, Schließen des ersten und des zweiten Reihenschalters und Schließen der zweiten Schaltereinrichtung zu einer ersten Zeit, um das Signal mit der ersten Polarität an das Herz zu liefern, und Entladen des Kondensators über die Leitungen auf eine Spannung, die der ersten Endamplitude entspricht;
Anlegen, an die Leitungen, einer zweiten verkürzten exponentiellen Wellenform mit einer zweiten Anfangsamplitude (V2), die im wesentlichen gleich einer Hälfte der ersten Anfangsamplitude (V1) ist, und einer zweiten Endamplitude (V3) und mit einer zweiten Polarität (64), die der mit der ersten Polarität entgegengesetzt ist, durch Umkehren der Polarität der Verbindung des Kondensators mit den Leitungen durch Öffnen des ersten und des zweiten Reihenschalters (122, 124) und der zweiten Schaltereinrichtung (100) zu einer zweiten Zeit nach der ersten Zeit, Schließen der dritten und der vierten Schaltereinrichtung zu einer dritten Zeit nach der zweiten Zeit, um das Signal mit der zweiten Polarität an das Herz zu liefern, wodurch der Kondensator auf eine Spannung entladen wird, die der zweiten Endamplitude (V3) entspricht, und Überbrücken der Ladung des Kondensators durch Nebenschluß zu einer vierten Zeit nach der dritten Zeit durch Öffnen der dritten Schaltereinrichtung während des Schließens des ersten Reihenschalter (122).

9. Verfahren nach Anspruch 8, wobei die zweite Anfangsamplitude gleich einem Wert zwischen vierzig und sechzig Prozent der ersten Anfangsamplitude ist.

## Revendications

1. Appareil implantable pour administrer une électrothérapie à un coeur, comprenant:
un moyen de reconnaissance (36) sensible à un signal électrique provenant du coeur pour déterminer lorsque le coeur a besoin d'une électrothérapie;
un moyen de stockage d'énergie capacitif (74) pour stocker une énergie électrique à appliquer au coeur;
un moyen de source d'énergie (72) pour appliquer ladite énergie électrique sur ledit moyen de stockage d'énergie;
un moyen de conducteur (26, 33) pour conduire ladite énergie électrique stockée depuis ledit moyen de stockage d'énergie (74) jusqu'au coeur; et
un moyen de connexion (100, 122, 124, 148, 156) pour connecter ledit moyen de conducteur (26, 33) selon une première polarité pour administrer un premier choc et selon une seconde polarité opposée à ladite première polarité pour administrer un second choc, après administration dudit premier choc, ledit moyen de connexion incluant:
un premier moyen de commutateur (122, 124) incluant des premier et second commutateurs (122, 124) en série pour connecter une première borne (+) dudit moyen de stockage d'énergie (74) à une première connexion (33) au coeur, un moyen de conduction unidirectionnelle (174) pontant le second commutateur série;
un second moyen de commutateur (100) pour connecter une seconde borne (-) dudit moyen de stockage d'énergie (74) à ladite seconde connexion (26) au coeur;
un troisième moyen de commutateur (156) pour connecter ladite première borne (+) dudit moyen de stockage d'énergie (74) à ladite seconde connexion (26) au coeur;
un quatrième moyen de commutateur (148) pour connecter ladite seconde borne (-) dudit moyen de stockage d'énergie (74) à un point (A) entre les premier et second commutateurs; et
un moyen de cadencement (49) pour fermer lesdits premier et second commutateurs série (122, 124) et ledit second moyen de commutateur (100) à un premier instant, pour ouvrir lesdits premier et second commutateurs série (122, 124) et ledit second moyen de commutateur (100) à un second instant après ledit premier instant, pour fermer ledit troisième moyen de commutateur (156) et ledit quatrième moyen de commutateur (148) à un troisième instant après ledit second instant et pour ouvrir ledit troisième moyen de commutateur (156) tout en fermant ledit premier commutateur série (122) à un quatrième instant après ledit troisième instant de manière à terminer ainsi ledit second choc de seconde polarité, ledit premier commutateur série (122) et ledit quatrième moyen de commutateur (148) étant conçus pour décharger ledit moyen de stockage d'énergie capacitif (74) audit quatrième instant.

2. Appareil selon la revendication 1, dans lequel ledit moyen de stockage d'énergie capacitif (74) est un unique condensateur (39).

3. Appareil selon la revendication 1, dans lequel le moyen de stockage d'énergie capacitif (74) inclut deux condensateurs (74A, 74B) connectés en série, lesdits deux condensateurs présentant des valeurs de capacité égales.

4. Appareil selon la revendication 1, comprenant en outre un moyen de réduction de tension (144) pour réduire la tension aux bornes dudit quatrième moyen de commutateur à un cinquième instant, ledit cinquième instant étant après ledit second instant et avant ledit troisième instant.

5. Appareil selon la revendication 4, dans lequel ledit moyen de réduction de tension (144) décale ladite tension depuis les bornes dudit quatrième moyen de commutateur (148) sur les bornes dudit premier commutateur série (122).

6. Appareil selon la revendication 1, comprenant en outre une première résistance (150) connectée aux bornes dudit premier commutateur série (122), un cinquième moyen de commutateur (144) comportant une première borne connectée à la seconde borne (-) dudit moyen de stockage d'énergie (74) et une seconde résistance (152) connectant une seconde borne dudit cinquième moyen de commutateur et un point de connexion entre ledit premier commutateur série (122) et ledit second commutateur série (124).

7. Appareil selon la revendication 6, comprenant en outre un condensateur (154) connecté entre ledit point de connexion et ladite seconde borne (-) pour ledit moyen de stockage d'énergie (74).

8. Procédé de fonctionnement d'un appareil implantable pour appliquer un courant électrique à un coeur au moyen de première et seconde connexions conductrices (33, 26) connectées électriquement à des électrodes associées au coeur, l'appareil incluant un condensateur (74) et un moyen de connexion de commutateur incluant:
un premier moyen de commutateur incluant des premier et second commutateurs (122, 124) en série pour connecter une première borne du condensateur à la première connexion conductrice (33), un moyen de conduction unidirectionnelle (174) pontant le second commutateur série;
un second moyen de commutation (100) pour connecter une seconde borne du condensateur (74) à la seconde connexion conductrice (26);
un troisième moyen de commutateur (156) pour connecter la première borne du condensateur à la seconde connexion conductrice (26);
un quatrième moyen de commutateur (148) pour connecter la seconde borne du condensateur à un point (A) entre les premier et second commutateurs,
dans lequel le procédé comprend les étapes de:
application d'une première forme d'onde exponentielle tronquée d'une première polarité (58) présentant une première amplitude de début (V1) et une première amplitude de fin (V2) sur les première et seconde connexions conductrices en chargeant le condensateur jusqu'à une tension correspondant à la première amplitude de début, en fermant les premier et second commutateurs série et en fermant le second moyen de commutateur à un premier instant pour produire le signal de première polarité sur le coeur et pour décharger le condensateur par l'intermédiaire des connexions jusqu'à une tension correspondant à la première amplitude de fin;
application sur les connexions d'une seconde forme d'onde exponentielle tronquée présentant une seconde amplitude de début (V2) sensiblement égale à la moitié de la première amplitude de début (V1) et une seconde amplitude de fin (V3) et présentant une seconde polarité (64) opposée à la première polarité, en inversant la polarité de connexion du condensateur sur les connexions, en ouvrant les premier et second commutateurs série (122, 124) et le second moyen de commutateur (100) à un second instant après le premier instant, en fermant les troisième et quatrième moyens de commutateur à un troisième instant après le second instant pour produire le signal de seconde polarité sur le coeur pour ainsi décharger le condensateur jusqu'à une tension correspondant à la seconde amplitude de fin (V3) et en dérivant la charge du condensateur à un quatrième instant après le troisième instant en ouvrant le troisième moyen de commutateur tout en fermant le premier commutateur série (122).

9. Procédé selon la revendication 8, dans lequel ladite seconde amplitude de début est comprise entre 40 et 60% de ladite première amplitude de début.
